# EUROPEAN PATENT APPLICATION

(11) **EP 3 354 197 A1**
(43) Date of publication of application: **01.08.2018**
(21) Application number: 17153752.5
(22) Date of filing: 30.01.2017
(51) Int. Cl.: A61B 5/044, A61B 5/046

(54) **METHOD AND SYSTEM FOR IDENTIFYING POTENTIAL ATRIAL FLUTTER AREAS FOR MEDICAL DECISION SUPPORT**

(71) Applicant: Karlsruher Institut für Technologie, 76131 Karlsruhe (DE); Städtisches Klinikum Karsruhe gGmbH, 76131 Karlsruhe (DE)
(72) Inventor: Oesterlein, Tobias, 76227 Karlsruhe (DE); Dössel, Olaf, 76646 Bruchsal (DE); Schmitt, Claus, 69121 Heidelberg (DE); Luik, Armin, 76187 Karlsruhe (DE)
(74) Representative: Bittner, Peter

(57) **Abstract**

A decision support computer system, computer-implemented method and computer program product for supporting diagnostic analysis of potential ablation areas to cure atrial flutter. An interface component (110) receives an electrical reference signal (RS) generated by a reference sensor (CSCS). The reference signal (RS) provides a time reference for electrical activity of one or more atria of a patient wherein the time interval between two subsequent electrical activities is referred to as basic cycle length (BCL). Further, it receives a plurality of electrical signals (P1 to Pi) generated by one or more further sensors (S1 to Sn) wherein the plurality of electrical signals (P1 to Pi) relate to a plurality of locations in the one or more atria. The plurality of locations includes locations different from the location of the reference sensor (CSCS). A signal analyzer component (120) determines, for each signal of at least a subset of the received signals originating from locations within a selected area of the one or more atria, when electrical activity occurs. Further, it determines, for a plurality of time points within the basic cycle length, size values for respective areas of active tissue by aggregating the size of surface elements associated with the locations where the respective signals show activity at the respective time points. A visualizer component (130) sets a visual property value for at least a subset of the surface elements wherein the subset includes surface elements associated with locations defining a particular area of active tissue which is smaller than active tissue areas associated with other locations. The visual property value indicates, in a visualization of the selected area, the particular area of active tissue as a potential ablation area to cure atrial flutter.

## Description

### Technical Field

The present invention generally relates to electronic data processing, and more particularly, relates to methods, computer program products and systems for identifying potential atrial flutter maintaining areas based on clinical sensor data.

### Background

Atrial flutter can frequently be observed as either index arrhythmia or as organized tachycardia following the ablation of atrial fibrillation (AFib). In the latter case, atrial flutter (AFlut) often is resistant to pharmacological treatment and cardioversion, but as well seen as intermediate step before normal sinus rhythm (SR) is achieved. In both cases, however, numerous studies have already demonstrated consistent results with respect to the underlying mechanisms and their anatomical locations. This understanding and the resulting treatment decisions have led to success rates in the order of 80 to 100% when catheter ablation is applied to terminate AFlut. The understanding of the patient-specific variant, however, is the central prerequisite of successful ablation. Details regarding the medical foundations can be found in the following references: "A classification of atrial flutter and regular atrial tachycardia according to electrophysiological mechanisms and anatomical bases; a Statement from a Joint Expert Group from The Working Group of Arrhythmias of the European Society of Cardiology and the North American Society of Pacing and Electrophysiology (by Saoudi, N.; Cosio, F.; Waldo, A.; Chen, S. A.; lesaka, Y.; Lesh, M.; Saksena, S.; Salerno, J.; Schoels, W.; European Heart Journal; 2001; 22; 1162-1182)"; "Atrial tachycardia after ablation of persistent atrial fibrillation: identification of the critical isthmus with a combination of multielectrode activation mapping and targeted entrainment mapping; (by Patel, A. M.; d'Avila, A.; Neuzil, P.; Kim, S. J.; Mela, T.; Singh, J. P.; Ruskin, J. N.; Reddy, V. Y.; 2008; Circulation. Arrhythmia and Electrophysiology; 1: 14-22)"; and "Characterization, Mapping and Ablation of Complex Atrial Tachycardia: Initial Experience with a Novel Method of Ultra High-Density 3D Mapping; (by Schaeffer, B.; Hoffmann, B. A.; Meyer, C.; Akbulak, R. O.; Moser, J.; Jularic, M.; Eickholt, C.; Nuhrich, J. M.; Kuklik, P.; Willems, S.; 2016; Journal of Cardiovascular Electrophysiology; 27: 1139-1150)".

Currently, this diagnostic process is primarily based on the experience of the physician. Although comprehensive electrogram data can be acquired from the complete atrium using an electro-anatomical mapping system (EAMS) with multi-polar mapping catheters, only two automatic analysis techniques are known. First, the maximum bipolar voltage of electrograms (EGMs) is evaluated within one cycle. Regions which exhibit voltage values below a certain threshold are considered scar, potentially being part of an anatomical obstacle. Typical threshold values for atrial myocardium are in the range of 0.5mV for zones of low voltage and 0.05mV for dense scar. Second, the local activation time (LAT) is annotated in each signal. After all signals are synchronized, the LAT reflects the global excitation pattern. This static image allows to gain a first impression of the course of atrial activation.

However, a number of additional features are assessed by physicians in the diagnostic process and typically evaluated manually by visual inspection. Considering the analysis of individual signals, these comprise the presence of double potentials, and evaluation of fractionation and duration of activity in each signal. From a multichannel point of view, additional features can be determined, like the mid-diastolic activity, area-based cycle length coverage and the critical isthmus. In addition to the references above by Patel et al. and Schaeffler et al., further medical details regarding the additional features are disclosed in the references: "A deductive mapping strategy for atrial tachycardia following atrial fibrillation ablation: importance of localized reentry; (by Jais, P.; Matsuo, S.; Knecht, S.; Weerasooriya, R.; Hocini, M.; Sacher, F.; Wright, M.; Nault, I.; Lellouche, N.; Klein, G.; Clementy, J.; Haissaguerre, M.; 2009; Journal of Cardiovascular Electrophysiology; 20; 480-491)", "Treatment of macro-re-entrant atrial tachycardia based on electroanatomic mapping: identification and ablation of the mid-diastolic isthmus; (by De Ponti, R.; Verlato, R.; Bertaglia, E.; Del Greco, M.; Fusco, A.; Bottoni, N.; Drago, F.; Sciarra, L.; Ometto, R.; Mantovan, R.; Salerno-Uriarte, J. A.; 2007; Europace : European Pacing, Arrhythmias, and Cardiac Electrophysiology : Journal of the Working Groups on Cardiac Pacing, Arrhythmias, and Cardiac Cellular Electrophysiology of the European Society of Cardiology; 9; 449-457)", and "Selection of Critical Isthmus in Scar-Related Atrial Tachycardia Using a New Automated Ultrahigh Resolution Mapping System; (by D. G. Latcu, S. - S. Bun, F. Viera, T. Delassi, M. El Jamili, A. Al Amoura, N. Saoudi; 2017; Circulation. Arrhythmia and Electrophysiology; 10; online ahead of print".

The flood of clinical data measured by corresponding sensors is overwhelming for any physician and does not appropriately support a physician in his/her effort in diagnosing atrial flutter types and locations for a patient.

### Summary

Therefore, there is a need to provide a diagnostic decision support solution with a corresponding method to assess these parameters automatically in order to support a medically trained human (e.g., a physician) in understanding the flutter circuit for improved diagnostic decision making as a basis for successful ablation.

This technical problem is solved by the features of a computer system, a computer-implemented method and a computer program product as disclosed in the independent claims.

In one embodiment, a decision support computer system for supporting diagnostic analysis of potential ablation areas to cure atrial flutter includes an interface component to receive an electrical reference signal generated by a reference sensor. Typically, a coronary sinus catheter sensor (CSCS) is used as a reference sensor. However, a reference sensor may also be placed at a location different from the coronary sinus in the patient's atria. In one embodiment, an ECG sensor may also be used as a reference sensor. The reference signal provides a time reference for electrical activity of one or more atria of a patient wherein the time interval between two subsequent electrical activities is referred to as basic cycle length (BCL). Time reference, as used herein, means that any delay times of signals measured by the further sensors at particular locations are measured in relation to the signal measured by the reference sensor. The interface further receives a plurality of electrical signals generated by one or more further sensors wherein the plurality of electrical signals relate to a plurality of locations in the one or more atria wherein the locations include locations different from the location of the reference sensor.

In one embodiment, the reference sensor may provide a position reference. Position reference, as used herein, means that the position of each further sensor is known relative to the position of the reference sensor. Often, the CSCS is used as the position reference. Typically, the locations of a few sensors in the heart are determined via magnet coils mounted at the tip of the catheter and underneath the patient whereas the other sensors are located using the principle of potential divider. Once the position of the reference sensor is determined the positions of other sensors are known relative to the position of the reference sensor. If the patient moves, the relative positions can be determined because the movement of the reference sensor is detected. It is also possible to determine the movement of the patient by other means. For example, the movement may be detected by a camera system or by ultrasonic sensors. The detected movement can then be used to re-compute the sensor locations by compensating the movement accordingly. In these implementations, the position reference function of the reference sensor is optional. Further, the interface receives a plurality of electrical signals generated by one or more further sensors wherein the plurality of electrical signals relate to a plurality of locations in the one or more atria. The plurality of locations is different from the location of the reference sensor (CSCS). In other words, the electrical signals received by the one or more further sensors illustrate how the excitation wave propagates through the atrium of a patient before and after passing the coronary sinus as detected by the reference sensor. The signals may be received by the interface directly from the sensors, thus enabling a live monitoring of the atria activity or it may be retrieved from a database which stores historic measurement data of the sensors.

Further, a signal analyzer component of the system determines, for each signal of at least a subset A of the received signals originating from locations within a selected area of the one or more atria, when electrical activity occurs. Typically, in case of atrial flutter, an electrical excitation wave is propagating through, and thus depolarizing, the tissue of the atrium. The surface of the atrium which is depolarizing at a particular point in time is referred to as the area of active tissue at this point in time. Dependent on the size of this area a plurality of the received signals corresponding to respective locations in the patient's atrium can show activity at the same time (simultaneous activity). The atrium is typically visualized for the medically trained person through a 3D graphical object. Typically, a 3D object surface is generated using a plurality of surface elements (e.g., polygons such as triangles or other shapes). For optimized processing, the mesh surface elements can be converted between different forms (triangles, tetrahedrons, etc.) or increased or decreased in density by increasing or reducing the number of surface elements. Each surface element can be assigned to a respective sensor location. For example, the respective sensor can be defined as the closest sensor in space. The signal analyzer then determines, for a plurality of time points within the basic cycle length, size values for respective areas of active tissue by assessing the aggregate size of the surface elements associated with the locations where the respective signals of subset A show activity at the respective time points. In other words, for the time points within the basic cycle length where corresponding sensor data has been sampled, the size value for respective areas of active tissue can be computed by adding up the size of surface elements associated with the locations showing activity at the respective time points. Alternatively, an average surface element size can be determined for a plurality of surface elements and the average size can be multiplied by the number of surface elements associated with the respective active area locations to assess the size of the areas of active tissue. It may also be possible that a smoothening operation, such as for example low-pass filtering, is performed on the resulting curve of area of active tissue (AAT) to smoothen it. Other smoothening algorithms may be used instead. The selected area typically corresponds to one or more atria of a patient but may be modified by a medically trained person or by the signal analyzer component. For a reentrant tachycardia mechanism, this area can e.g. be restricted to the path of excitation, excluding the areas which merely represent a dead end running into an anatomical obstacle.

The system further has a visualizer component configured to set a visual property for at least a subset of the surface elements. The subset includes surface elements associated with locations identifying a particular area of active tissue which is smaller than active tissue areas associated with other locations. In other words, tissue which is active when the AAT size value is minimal or at least smaller than the size value at other time points can be highlighted. The visual property is selected to indicate, in a visualization of the selected area, the particular area of active tissue as a potential ablation area to cure atrial flutter. It is a goal in atrial flutter treatment to restrict ablation to the smallest possible area which can cure the atrial flutter and optimizes cardiac function after restoring sinus rhythm by considering patient specific anatomical variations. Thus, it is advantageous for the medically trained person to receive one or more suggestions from the system for such potential ablation areas which are smaller than other parts of the atrium affected by the atrial flutter activity. The medically trained person can then make a decision for treatment of a particular area based on this information. For example, in case of a focal source atrial flutter the potential ablation area corresponds to a spot defining the approximate origin of the focal source. In case of a micro-reentry atrial flutter the potential ablation area corresponds to a bridging area between the approximate center of the micro-reentry affected area and the closest anatomical obstacle. In case of a macro-reentry atrial flutter the potential ablation area corresponds to the critical Isthmus within the macro-reentry affected area.

The visualization of the respective areas can be achieved by any suitable visual property setting for the corresponding surface elements. For example, the visual property value may represent particular color for highlighting the respective surface elements, a particular texture for highlighting the respective surface elements, or a particular animation mode for highlighting the respective surface elements.

In one embodiment, the particular area of active tissue size is computed as the minimal area of active tissue at a respective time point within the basic cycle length, the respective visual property value being indicative of the minimal area. In this embodiment, all surface elements associated with sensor locations of subset A showing activity at the point in time where the area of active tissue size reaches its minimum value during the basic cycle length may receive the same indicative setting of the visual property (e.g., a particular color, a particular pattern, a particular blinking mode, etc.). All other surface elements receive visual property values different from the indicative setting. In this embodiment only one potential ablation area is indicated and therefore suggested to the medically trained person who needs finally to decide whether the suggested indicated area is appropriate for the therapy.

In some cases it may be advantageous to present a plurality of alternative potential ablation areas. For example, there may be more than one area which is small enough for a successful ablation operation. Of course, only one of them is the smallest but the others may be equally suited for the intervention when considering additional patient specific information. In this case, the medically trained person is better supported in her decision making when multiple options of similar quality are presented to select from. This is achieved by an embodiment, where the particular area of active tissue is computed by computing statistical measures for the size values of active tissue areas measured during the activity period of a respective signal. The respective visual property value is thereby indicative of the statistical measure value characterizing the active tissue area size value during the activity period. In other words, in this embodiment, for each signal location a visual property value is computed which represents the statistical measure of the AAT size values measured during the activity time interval. Statistical measures can be determined by various statistical methods, such as for example, arithmetic averaging, median, mode, or first 10-quantile. Other statistical methods may also be applicable. That is, small areas of active tissue which are not the smallest one are not filtered out in this embodiment but visualized with a different but similar visual property as the minimal area. For example, besides averaging, the sizes of active tissue areas measured during the activity period of a respective signal can also be assessed by computing the median, mode or quantile. In one embodiment, the 10% quantile of active tissue areas observed within the activity period of a respective signal can be determined as visual property value. To avoid unnecessary visual coding (e.g., color coding, pattern coding, etc.) for areas which are not of interest at all as a potential ablation area, only such surface elements may be visually coded which belong to sensor locations associated with areas of active tissue below a threshold value. Such a threshold value may be predefined or may be computed at runtime, e.g. as a percentage of the maximal area of active tissue during the basic cycle length, or as a percentage surcharge on the minimal area of active tissue.

Current approaches to identify the critical part of the atrial flutter mechanism are based on visual inspection. The LATs of recorded signals are compared to the cumulative effect of atrial depolarization as indicated by the P-wave from the body-surface electrocardiogram. Signals which are recorded at the critical site of the flutter circuit are expected to show a LAT which is located temporarily between two consecutive P-waves. This measure is chosen, as the P-wave represents the best available surrogate for the atrial depolarization when no intra-atrial measurement data are available. In turn, the active tissue area as described herein is expected to be a more reliable indicator for the true amount of atrial depolarization. From electromagnetic field theory, the P-wave is formed by projecting the electrical field vector as generated by the atrial depolarization on the axes spanned by the recorded electrodes of the surface ECG. Directly assessing the active atrial tissue avoids this projection. Details about this current approach can be found in the above cited De Ponti reference.

It may be advantageous for the decision support of the medically trained person to know which atrial flutter type is present in the atrium. Therefore, to solve this problem, in one embodiment, the signal analyzer component may further determine, for each signal of at least a subset B (which can be part of subset A) of the received signals, an active interval when electrical activity occurs, and a resting interval when no electrical activity occurs. The analyzer may then compute, based on the determined intervals, a subset cycle length coverage as the ratio of duration of activity (DoA) and the basic cycle length wherein the duration of activity includes the active intervals for the entire subset B of signals. Electrical activity hereby is a consequence of the depolarization of cardiac tissue close to the sensor. No electrical activity occurs when neighboring tissue is in rest and does not depolarize. Thus the electrical activity reflects changes of the trans-membrane voltage of adjacent myocardial cells. For example, the selected area associated with subset B may correspond to an atrium. The selection may be predefined (e.g., the right atrium or the left atrium) or it may be received by the system via an appropriate user interface from a user of the system. For example, a physician may use a pointing mechanism (e.g., by using a mouse, a touch screen, or the like) to indicate selected areas of interest in a graphical visualization of the patient's heart. In other words, the physician may select both atria, only one atrium, or just a part of an atrium as area of interest for the later diagnostic analysis. Further, the signal analyzer may compute, based on the determined intervals (active intervals and resting intervals), a subset cycle length coverage as the ratio of duration of activity and the basic cycle length. Thereby, the duration of activity includes the active intervals for the entire subset B of signals. In other words, the duration of activity corresponds to one or more intervals which result from superposing all active intervals detected by the further sensors located within the selected area associated with subset B. In this embodiment, the visualizer component of the system can indicate to the medically trained human the selected area associated with subset B (e.g., right or left atrium) as an area including a potential focal source if the subset cycle length coverage is smaller than a predefined ratio. If the subset cycle length coverage is equal or above the predefined ratio, the selected area is indicated as an area including a potential re-entry. The indication may be implemented through visualization parameters setting a predefined color or pattern for the visualization of the identified atrial flutter type. "Focal source" and "re-entry" are the major atrial flutter types as they are known by the medically trained user of the system. For example, when a potential focal source is determined in the selected area, the selected area can be visualized to the medically trained person in a first color or using a first pattern associated with the atrial flutter type "focal source". When a potential re-entry is determined in the selected area, a second color or second pattern may be used which is associated with the atrial flutter type "re-entry".

A first indicator for the nature of the underlying tachycardia mechanism is given by the amount of cycle length which can be annotated when all recorded signals are assessed in conjunction. Reentry is typically suspected as mechanism for atrial flutter if activation times for at least 85% - 95% of the BCL (i.e., predefined ratio: 95%) can be successfully mapped. For each moment of the covered time, accordingly, an EGM can be found somewhere in the atrium which exhibits activity and whose local activity time can be annotated. Inversely, no activity can be detected for less than 5% -15% of the cycle length. Given a sufficiently high-density of intra-cardiac measurements, the atrium is suspected to be in rest during this inactive time, excluding the presence of a re-entrant source and endorsing a truly focal source. The coverage of macro re-entry activation was shown to be about 95.9±4.3% (range 90% to 100 %) in clinical mapping studies (cf., the above cited De Ponti reference). This is one important indicator that can be assessed when all recorded electrograms are analyzed jointly.

The buffer of 15% is frequently applied since some endocardial aspects of the flutter cycle may not be reachable during mapping, or the flutter may propagate on the epicardial aspect of the atrium. Thus the excitation wave cannot be observed at these locations and no LAT can be annotated. In addition, also highly fractionated potentials are assigned just one LAT value, despite the fact that they may cover significant parts of the cycle length. Studies have shown prolonged activation at the critical isthmus lasting mean durations of 200±80 ms, with individual potentials having duration of 360 ms as illustrated in "Flutter localized to the anterior left atrium after catheter ablation of atrial fibrillation (by Jais, P.; Sanders, P.; Hsu, L.-F.; Hocini, M.; Sacher, F.; Takahashi, Y.; Rotter, M.; Rostock, T.; Bordachar, P.; Reuter, S.; Laborderie, J.; Clementy, J.; Haissaguerre, M.; 2006; Journal of Cardiovascular Electrophysiology; 17; 279-285)". This demonstrated a situation in which the annotation of LAT will not lead to optimal results. Instead, an activity based approach is suggested in the following.

Although the annotation of LAT may not lead to optimal results, in one embodiment, a predefined LAT may be used to define a surrogate for the activity. Therefore, a time window between 10 milliseconds and 300 milliseconds (advantageously between 20ms and 30ms) around the LAT may be defined as active interval. This may be done independently of assessing the signal morphology, and thus not reflect the true activity. However, it may be used as an initial interval length which may already lead to results reflecting the general flutter type or allowing to define active tissue areas. For example, in this embodiment, the active interval (i.e. the predefined window) may be centered around a particular point in time where the respective signal shows activity. The particular time point can be the maximum or the minimum of the signal amplitude during a basic cycle length, or it may be the maximum of the absolute value of the time derivative of the signal. Typically, the activity of the signal swings first to a maximum value of the signal amplitude and then to a minimum value of the signal amplitude before turning back to a value close to zero (resting interval). Between the time points with maximum and minimum values of the signal amplitude the absolute value of the time derivative reaches its maximum. The predefined window may be centered around any one of these time points.

In one embodiment, the system can provide additional information regarding the type of atrial flutter re-entry. This flutter type includes two sub-types: micro-re-entry and macro-re-entry. Both atrial flutter types require different treatment of the patient. Therefore, it is advantageous if the medically trained person is provided with the additional information about the subtype of a potential re-entry. In this embodiment, once a potential re-entry is detected in the selected area, the signal analyzer component further can select a plurality of sub-areas within the originally selected area wherein the sub-areas have a size which is smaller than the size of the selected area, and wherein each sub-area has an overlapping area with at least one further sub-area. For example, the sub-areas may have a circular shape or another geometric shape (e.g., square, rectangle, triangle, etc.) which is appropriate to sub-divide the selected area into evenly spread sub-areas with overlapping areas between two neighboring sub-areas.

The signal analyzer can then compute for each sub-area the respective sub-area cycle length coverage. This computation is performed in an analogous manner to the computation of the sub-set cycle length coverage. However, instead of using the subset of signals originating from locations within the selected area for determining the active intervals and resting intervals only signals originating from locations within the respective sub-area are used for the computation. In other words, for the computation of a particular sub-area cycle length coverage the corresponding ratio of duration of activity and the basic cycle length takes into account the active intervals for the signals originating from a location within the particular sub-area.

Because of the higher spatial granularity of the sub-area cycle length coverages, there may be two or more neighboring sub-areas with different sub-area cycle length coverage values leading to a conflict situation of the respective overlapping area. To solve this problem with this embodiment, for each overlapping area with conflicting sub-area cycle length coverages from at least two overlapping sub-areas, one or more predefined conflict resolution rules are applied to determine a respective overlapping area cycle length coverage based on the conflicting sub-area cycle length coverages. For example, any one of the following conflict resolution rules may be used by the signal analyzer: assigning the conflicting sub-area cycle length coverage with the highest value to the respective overlapping area cycle length coverage; assigning the average value of the conflicting sub-area cycle length coverages to the respective overlapping area cycle length coverage; and assigning a weighted average value of the conflicting sub-area cycle length coverages to the respective overlapping area cycle length coverage.

In the case of the atrial flutter type "focal source" the duration of activity is approximately the same in all sub-areas. However, the origin of the focal source can be determined to be in the sub-area where the activity occurs first in time. In other words, the sub-area showing the earliest activity can be proposed to the physician as a potential target for later ablation.

Further, in this embodiment, the visualizer component can indicate sub-areas and overlapping areas with respective cycle length coverages equal or above an atrial flutter type threshold as areas including a potential micro re-entry, and further indicate sub-areas and overlapping areas with respective cycle length coverages below the atrial flutter type threshold as areas not including a potential micro re-entry. If no sub-area or overlapping area indicates the presence of a micro re-entry, a conclusion for the presence of a macro re-entry can be drawn. The indication at the level of sub-areas may be implemented through visualization parameters setting a predefined color or pattern associates with the respective atrial flutter type in a similar way as for the selected areas.

The spatial resolution regarding the identification of potential locations comprising an atrial flutter source depends on the size and shape of the sub-areas. In one embodiment, the sub-areas have a circular shape and the overlapping areas between two overlapping circles account for 1/5 to 1/3 of the sub-area diameter. In an alternative embodiment, the sub-areas have a circular shape with a diameter between 2 cm and 4 cm, and the circle centers of two neighboring sub-areas have a distance between 0,5 cm and 1,5 cm. In another embodiment, the sub-areas have a square shape with a diagonal between 2 cm and 4 cm, and the square centers of two neighboring sub-areas have a distance between 0,5 cm and 1,5 cm. In an alternative embodiment, the sub-areas have a square shape and the overlapping areas between two overlapping circles account for 1/5 to 1/3 of the sub-area diagonal. A person skilled in the art can use other geometric forms for the sub-areas and achieve a comparable coverage of the selected area with a comparable resolution by applying similar overlapping ratios as disclosed for circle and square shaped sub-areas.

In one embodiment, computing a subset cycle length coverage may include applying a Boolean OR operator to the determined activity and resting intervals of the respective signals so that any activity interval of any signal contributes to the duration of activity, and no activity in all signals contributes to the duration of resting.

In one embodiment, a computer-implemented method is provided for supporting diagnostic analysis of potential ablation areas to cure atrial flutter. The method may be executed by the previously disclosed system. The method includes: receiving an electrical reference signal generated by a reference sensor, the reference signal providing a time reference for electrical activity of one or more atria of a patient wherein the time interval between two subsequent electrical activities is referred to as basic cycle length; receiving a plurality of electrical signals generated by one or more further sensors wherein the plurality of electrical signals relate to a plurality of locations in the one or more atria, the plurality of locations comprising locations different from the location of the reference sensor; determining, for each signal of at least a subset of the received signals originating from locations within a selected area of the one or more atria, when electrical activity occurs; determining, for a plurality of time points within the basic cycle length, size values for respective areas of active tissue by assessing the aggregate size of surface elements associated with the locations where the respective signals show activity at the respective time points; setting a visual property for at least a subset of the surface elements wherein the subset includes surface elements associated with locations identifying a particular area of active tissue which is smaller than active tissue areas associated with other locations, to indicate, in a visualization of the selected area, the particular area of active tissue as a potential ablation area to cure atrial flutter.

In one embodiment, in case of a focal source atrial flutter, the potential ablation area may be visualized by highlighting a spot defining the approximate origin of the focal source. In one embodiment, in case of a micro-reentry atrial flutter the potential ablation area may be highlighted as a bridging area between the approximate center of the micro-reentry affected area and the closest anatomical obstacle. In one embodiment, in case of a macro-reentry atrial flutter the potential ablation area may be highlighted as the critical isthmus within the macro-reentry affected area.

In one embodiment, the method may further include computing the size value of the particular area of active tissue as the minimal area of active tissue at a respective time point within the basic cycle length wherein the respective visual property value is selected so that it is indicative of the minimal area (e.g., a particular color distinguishing the minimal area from the rest of the selected area.

In one embodiment, the method further includes computing the size value of the particular area of active tissue as statistical measure for the sizes of active tissue areas measured during the activity period of a respective signal, the respective visual property value being indicative of the statistical measure value. The statistical measure may be determined by statistical methods, such as for example, arithmetic averaging, median, mode, or first 10-quantile.

In one embodiment, the method may further include steps to provide suggestions for an atrial flutter type by: determining, for each of the signals of at least the subset, an active interval when electrical activity occurs, and a resting interval when no electrical activity occurs; based on the determined intervals, computing a subset cycle length coverage as the ratio of duration of activity (DoA) and the basic cycle length (BCL) wherein the duration of activity (DoA) includes the active intervals for the entire subset of signals; if the subset cycle length coverage is smaller than a predefined ratio then indicating the selected area as an area including a potential focal source, else indicating the selected area as an area including a potential re-entry.

In one embodiment, a computer program product is provided for supporting diagnostic analysis of potential ablation areas to cure atrial flutter. The computer program product is loaded into a memory of a computing device/system as disclosed herein and executed by at least one processor of the computing device. This causes the computing device to execute the steps of the above disclosed computer-implemented method thereby implementing the features of the system as disclosed herein.

In one embodiment, the method may further include: if the selected area includes a potential re-entry, selecting a plurality of sub-areas within the originally selected area wherein the sub-areas have a size which is smaller than the size of the selected area, and wherein each sub-area has an overlapping area with at least one further sub-area; computing for each sub-area the respective sub-area cycle length coverage; for each overlapping area with conflicting sub-area cycle length coverages from at least two overlapping sub-areas, applying one or more predefined conflict resolution rules to determine a respective overlapping area cycle length coverage based on the conflicting sub-area cycle length coverages; indicating sub-areas and overlapping areas with respective cycle length coverages equal to or above an atrial flutter type threshold as areas including a potential micro re-entry. Optionally, the method may include indicating sub-areas and overlapping areas with respective cycle length coverages below the atrial flutter type threshold as areas including a potential macro re-entry. The presence of a macro re-entry can be concluded if no sub-area or overlapping area indicates the presence of a micro re-entry. As previously disclosed, the indication of the various atrial flutter types can be controlled by corresponding visualization parameter associated with the respective atrial flutter types. The visualization parameters may represent predefined different colors, patterns, animations, image depth in 3D visualizations, etc. for the various flutter types. When the system visualizes a particular atrial flutter type, the associated visualization parameter is used to render the respective area (selected area or sub-areas) in a graphical representation of the patient's atria.

For example, a predefined conflict resolution rule can include assigning the conflicting sub-area cycle length coverage with the highest value to the respective overlapping area cycle length coverage. In this example, the visualization parameter for the respective overlapping area equals the visualization parameter of the corresponding sub-area with the highest value.

For example, a predefined conflict resolution rule can include assigning the average value of the conflicting sub-area cycle length coverages to the respective overlapping area cycle length coverage. In this example, a visualization parameter for the overlapping area is computed based on visualization parameters of the sub-areas having conflicting cycle length coverages. For example, if the visualization parameter of a first sub-area indicates a red visualization and the visualization parameter of a second sub-area indicates a blue visualization, the visualization parameter of the overlapping area may result in a violet visualization. The average value may be computed based on RAL values of the respective colors. Other number values describing colors may be used instead. In case of using pattern based visualization parameters, in one example the first sub-area may have the visualization parameter horizontal hatch and the second sub-area may have a vertical hatch pattern. The average may be computed as the overlay of both hatch patterns resulting in a cross hatch. In the case of using animations (e.g., blinking of the respective area) the animation frequency may be the visualization parameter for averaging.

For example, a predefined conflict resolution rule can include assigning a weighted average value of the conflicting sub-area cycle length coverages to the respective overlapping area cycle length coverage. A person skilled in the art can adjust the conflict resolution rules for assigning average values by simply adding weighting factors which may emphasize, for example, the visualization parameter of the sub-area having the sub-area cycle length coverage with the highest value.

Further aspects of the invention will be realized and attained by means of the elements and combinations particularly depicted in the appended claims. It is to be understood that both, the foregoing general description and the following detailed description are exemplary and explanatory only and are not restrictive of the invention as described.

### Brief Description of the Drawings

FIG. 1 is a simplified block diagram illustrating an embodiment of a decision support computer system for supporting diagnostic analysis of potential ablation areas to cure atrial flutter;
FIG. 2 is a simplified flowchart of a computer-implemented method for supporting diagnostic analysis of potential ablation areas to cure atrial flutter according to an embodiment of the invention;
FIG. 3 is a schematic illustration of a human heart with the right atrium showing a micro-reentry atrial flutter;
FIG. 4 is a schematic illustration of an atrium with representations of sensor locations;
FIG. 5 illustrates sensor signals received from different locations of a patient's atrium with activity intervals;
FIG. 6A is a graph illustrating the area of active tissue over time during the basic cycle length with averaged active tissue area values for the active periods of two sensors;
FIG. 6B is a graph illustrating the area of active tissue over time with the minimal area of active tissue during the basic cycle length;
FIG. 7 is a schematic illustration of an atrium with representations of sensor locations and an indication of a potential ablation area;
FIG. 8 shows a magnified portion of FIG. 7 including the potential ablation area with highlighted surface elements;
FIG. 9 shows two examples of active tissue graphs for a right and a left atrium based on real sensor data;
FIG. 10 shows an example of an atrium which is color coded according to one embodiment to indicate potential ablation areas;
FIG. 11 illustrates sensor signals received from different locations of a patient's atrium with aggregate activity periods during the basic cycle length;
FIG. 12 illustrates the duration of activity and the duration of resting for the determination of cycle length coverage;
FIG. 13 is a simplified illustration of overlapping sub-areas on a graphic representation of an atrium according to one embodiment of the invention; and
FIG. 14 is a diagram that shows an example of a generic computer device and a generic mobile computer device which may be used with the techniques described herein.

### Detailed Description

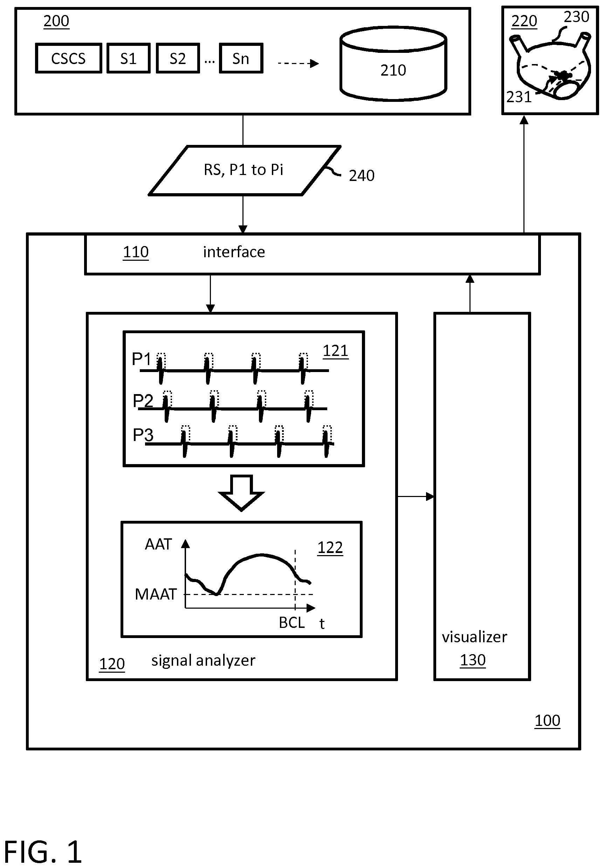

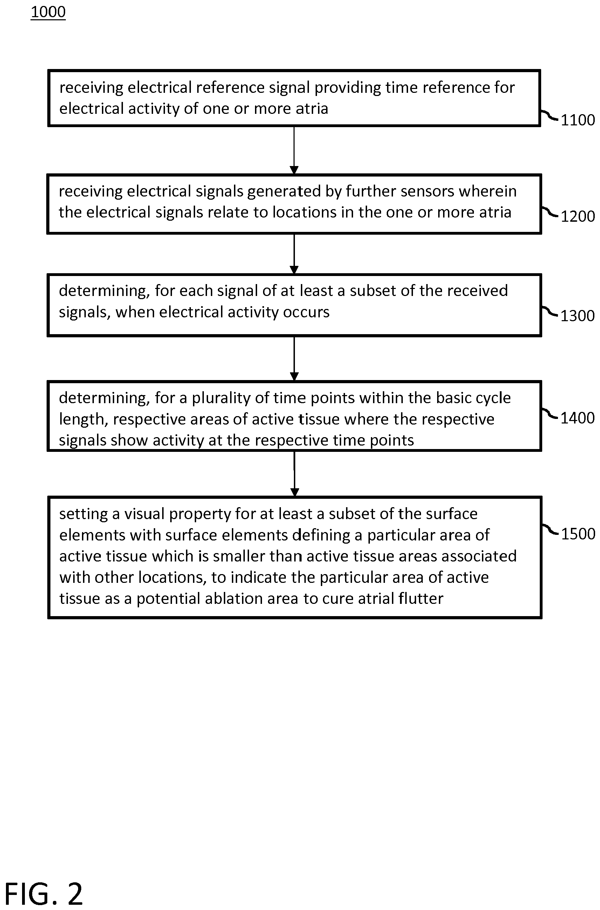

FIG. 1 is a simplified block diagram illustrating an embodiment of a decision support computer system 100 for supporting diagnostic analysis of potential ablation areas to cure atrial flutter. FIG. 2 is a simplified flowchart of a computer-implemented method 1000 for supporting diagnostic analysis of potential ablation areas to cure atrial flutter according to an embodiment of the invention. The functions of system 100 of FIG. 1 are discussed in the context of the method steps of method 1000 which are performed by the respective system components of system 100. Therefore, the following description refers to reference numbers of the FIGs. 1 and 2.

The system 100 includes an interface component 110 configured to receive 1100, 1200 data 240 from one or more external data sources 200. The external data sources may be sensors CSCS, S1 to Sn providing real time data about the electric activation of a patient's atria or it may be a data storage device 210 which provides historic (previously recorded or simulated) sensor data about the electric activation of the patient's atria.

The received data 240 includes an electrical reference signal RS generated by a reference sensor. Typically, a coronary sinus catheter sensor CSCS is used to measure the electric activity in a patient's atria at the location of the coronary sinus. This reference signal RS provides a position reference and a time reference for electrical activity of one or more atria of the patient. Thereby, the time interval between two subsequent electrical activities is referred to as basic cycle length (BCL).

The interface further receives 1200 a plurality of electrical signals P1 to Pi generated by one or more further sensors S1 to Sn wherein the plurality of electrical signals P1 to Pi relate to a plurality of locations in the one or more atria, the plurality of locations being different from the location of the reference sensor. Typically, such sensors are multi-polar mapping catheters which are widely used for electro-anatomical mapping systems EAMS. Examples of such sensors are described in detail in the references: "A multi-purpose spiral high-density mapping catheter: initial clinical experience in complex atrial arrhythmias; (by Jones, D. G.; McCready, J. W.; Kaba, R. A.; Ahsan, S. Y.; Lyne, J. C.; Wang, J.; Segal, O. R.; Markides, V.; Lambiase, P. D.; Wong, T.; Chow, A. W. C.; 2011; Journal of Interventional Cardiac Electrophysiology: an International Journal of Arrhythmias and Pacing; 31: 225-235)", and "Rapid high resolution electroanatomical mapping: evaluation of a new system in a canine atrial linear lesion model; (by Nakagawa, H.; Ikeda, A.; Sharma, T.; Lazzara, R.; Jackman, W. M.; 2012; Circulation. Arrhythmia and Electrophysiology; 5; 417-424)".

The system 100 further has a signal analyzer component 120. The signal analyzer 120 determines, for each signal P1, P2, P3 of at least a subset 121 of the received signals P1 to Pi when an electrical activity occurs at the location of the respective sensor. Thereby, the subset 121 includes such signals P1, P2, P3 which originate from locations within a selected area of the one or more atria. Typically, the selected area is one atrium (of the patient's heart) which is subject to the diagnostic analysis. The selected area may also relate to a portion of an atrium. The selection of the selected area may be received from the medically trained person performing the diagnosis or it may be predefined in the system 100. For example, in a first analysis the right atrium (or a portion) may be selected and in a second analysis the left atrium (or a portion) may be selected. Based on the activity intervals of the various signals in the subset 121, for each signal in the subset 121, the signal analyzer determines 1300 an active interval when electrical activity occurs. For example, the detection of activity may be performed by assessing the amplitude of the measured signal in that a window is shifted over the signal with the maximum and minimum amplitude being computed and the current window being identified as activity during an active interval if the difference between maximum and minimum values exceeds a certain threshold. In an alternative implementation, the detection of activity may be performed by assessing the instantaneous energy of the measured signal, in which a threshold can be used to distinguish active and inactive parts of the signal. The durations of the electrical activity of the signals P1, P2, P3 in subset 121 are illustrated by the rectangular shapes which are superposed to the signal curves.

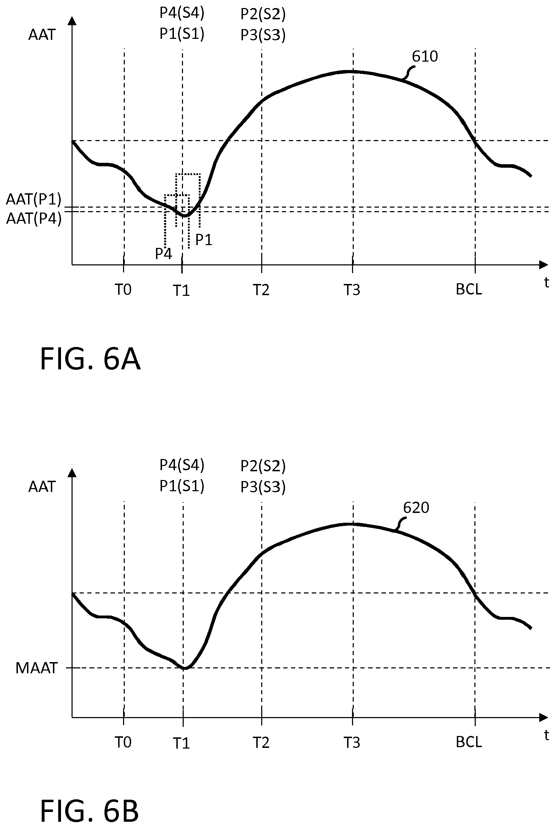

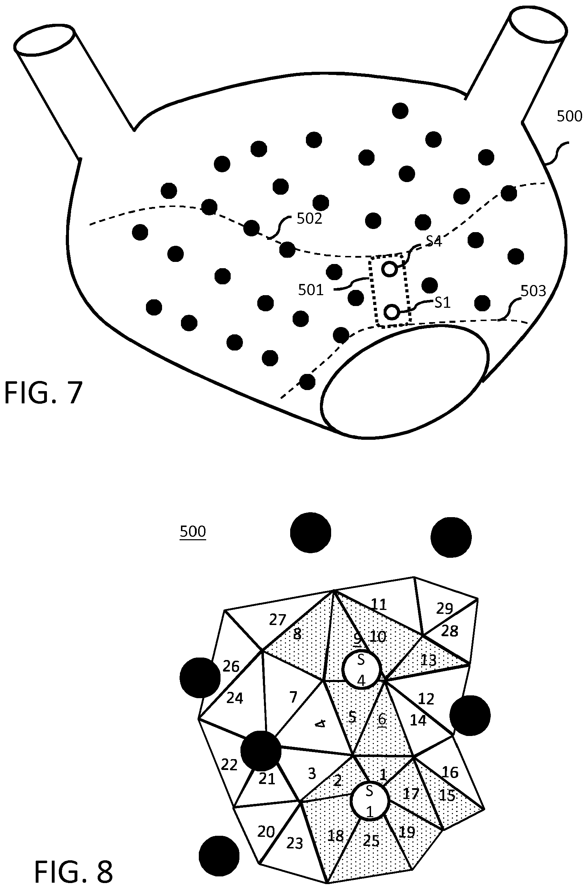

An active area component 122 of the signal analyzer 120 determines 1400, for a plurality of time points within the basic cycle length BCL, respective areas of active tissue AAT by aggregating the size of surface elements (e.g., by adding the size of the surface elements, counting the number of surface elements and multiply by an average size, etc.) associated with the locations where the respective signals show activity at the respective time points. FIG. 8 discusses in detail how surface elements can be associated with sensor locations. The AAT graph illustrated in FIG. 1 shows an example embodiment where a minimal area of active tissue MAAT is determined. This embodiment is discussed in detail in the context of FIG. 6B.

By using the above disclosed signal analysis the system 100 can compute one or more areas in the patient's atrium which are appropriate for a potential ablation operation. Such one or more potential ablation areas are then conveyed by the system 100 to the medically trained person to support the diagnosis and take a decision for the appropriate treatment of the patient. For this purpose, the system includes a visualizer component 130 which sets 1500 a visual property (e.g., a visual property selected from the earlier disclosed examples, such as a particular color for highlighting the respective surface elements, a particular texture for highlighting the respective surface elements, or a particular animation mode for highlighting the respective surface elements) for at least a subset of the surface elements. The subset includes surface elements associated with locations identifying a particular area of active tissue which is smaller than active tissue areas associated with other locations which are active at other time points. The particular area of active tissue is then indicated, in a visualization of the selected area, as a potential ablation area to cure atrial flutter. As explained earlier, it can be advantageous to highlight not only the smallest (minimal) area of active tissue but also other areas of active tissue which may be bigger but similar in size to the smallest area, and therefore, may represent potential ablation areas of similar quality.

As discussed above, visual properties, such as color, patterns or animations may be used to render a graphic representation 230 of the patient's atria on a display device 220 (e.g., communicatively coupled with the system 100 via interface 110) highlighting one or more areas of active tissue as potential ablation areas in such a way that the medically trained user of the system is provided with clear information about alternative options for ablation treatment. Any appropriate display technology (e.g., monitor, touch screen, etc.) may be used for the visualization of the potential ablation area(s) 231.

For example, in case of a focal source atrial flutter the potential ablation area typically corresponds to a spot defining the approximate origin of the focal source. For example, in case of a micro-reentry atrial flutter the potential ablation area corresponds to a bridging area between the approximate center of the micro-reentry affected area and the closest anatomical obstacle. For example, in case of a macro-reentry atrial flutter the potential ablation area corresponds to the critical isthmus within the macro-reentry affected area.

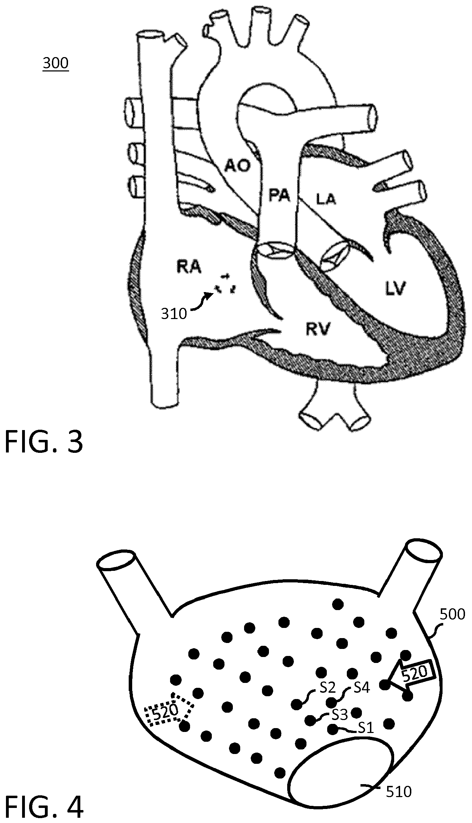

FIG. 3 is a schematic illustration 300 of a human heart with the right atrium RA showing a micro-reentry atrial flutter 310. The human heart includes the right and left atria RA, LA and the right and left ventricles RV, LV. The illustration also shows the aorta AO and the pulmonary aorta PA. In the right atrium RA the three arrows 310 representing a circular excitation wave illustrate the presence of a micro-reentry in the patient's right atrium RA.

FIG. 4 is a schematic illustration of an atrium 500 with representations of sensor locations S1, S2, S3, S4, etc. For convenience of illustration only the sensor locations S1 to S4 have a reference sign in the figure. The atrium 500 can be a right or a left atrium. The sensor locations are illustrated by black dots. The sensor locations do not necessarily correspond to locations where sensors are operated simultaneously. One sensor may have been moved to various sensor locations for taking measurements regarding the electrical activity at the respective location. In real world measurement scenarios up to about 12000 sensor locations may provide a high spatial resolution image of the electrical activity in the atrium. Measurements at different locations can be taken at different times but may be merged afterwards when computing the areas of active tissue because the electrical activation occurs periodically with a period length corresponding to the basic cycle length. Therefore, only the relative times of the activity intervals of the sensor signals within a basic cycle length are relevant for the determination of the potential ablation areas.

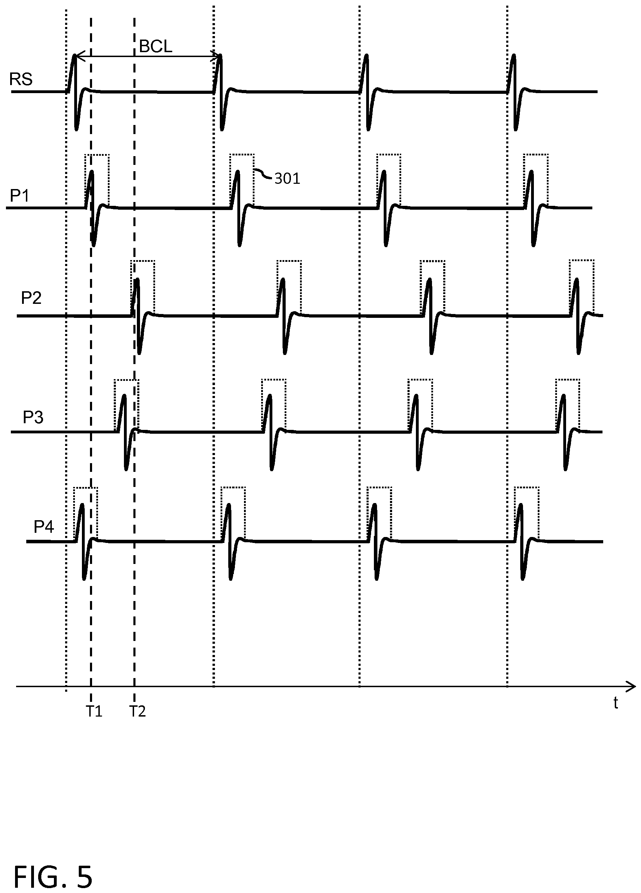

FIG. 5 illustrates sensor signals RS, P1 to P4 received from different locations of a patient's atrium with activity intervals reflecting activation for particular locations of the atrium. In the example, the reference signal RS is shown at the top providing the time reference for the signals P1 to P4 which are taken by sensors S1 to S4 (cf. FIG. 4) at locations different from the location of the reference sensor (not shown in FIG. 4). The reference signal RS illustrates the periodic activity observed at the coronary sinus. The time between two voltage amplitude peaks is defined as the basic cycle length BCL. Besides maxima in the voltage, also other characteristic morphological markers of the electrical signal, such as for example, minima, strongest gradient, and so forth can be used to assign the reference time. As the reference signal corresponds to an electrical signal which is generated by a human organ and not by a clock signal of a technical system, there may be small variability in the BCL length over time. This has been discussed in the Saoudi reference cited above. Continuously monitoring the CS catheter reference signal allows to compensate for such small variability in BCL by scaling the duration of the resulting active and inactive periods. For example, every 300 milliseconds a variance of +/- 15 milliseconds may occur (modulation of BCL caused by respiration). By comparing the activity intervals with the corresponding actual BCL, the computed subset cycle length coverage is automatically normalized with regards to the actual BCL. The actual BCL can be manually defined by the medically trained person or can be based on a median value of all measured actual BCL or it may be based on a mean value of all measured actual BCL.

The signal analyzer determines for each signal P1 to P4 the activity intervals for each cycle. In the example of FIG. 5 a cycle is indicated as the time between two of the vertical dotted lines. However, it is not relevant where a cycle starts and ends as long as the cycle length corresponds to BCL. The activity intervals of the respective signals are illustrated by the rectangular overlays. For simplicity, only the rectangular overlay 301 is shown with a reference number. The length of the resting interval of a cycle is defined as the difference between BCL and the length of the respective active interval of the signal.

In the example of FIG. 4 it is assumed that the atrium 500 (cf. FIG. 4) suffers from a macro-reentry flutter which causes an excitation wave 520 from the right to the left. Such an excitation wave of a macro-reentry is typically rotating around the opening 510 of the atrium (to the corresponding ventricle). That is, the excitation wave moves from left to right at the backside of the atrium (illustrated by dashed arrow 520) to re-appear at the right side on the front side (the illustrated view). Turning back to FIG. 5, sensor S4 measures the activity in P4 shortly before sensor S1 measures activity in P1. During a first overlapping time interval including time point T1 S1 and S4 measure activity simultaneously. When the wave propagates to the left, sensor S3 measures the activity in P3 shortly before sensor S2 measures the activity in P2. During a second overlapping time interval including time point T2 S3 and S2 measure activity simultaneously.

FIG. 6A shows a graph 610 illustrating the size of the area of active tissue AAT over time t during the basic cycle length BCL with averaged active tissue area size values AAT(P1), AAT(P4) for the active periods of the two sensors S1, S4, respectively. The overlapping activity intervals of P4 and P1 are again illustrated as subsequent rectangular shapes which include the time point T1. The AAT 610 shows a minimum at T1 during the BCL. The time points T0, T2, T3 are associated with larger areas of active tissue than T1. For example, T2 corresponds to the time point included in the overlap of the activity intervals (i.e. activity periods) of P2, P3. In this embodiment, the size of the particular area of active tissue is computed as statistical measure (in the example, the average values AAT(P4), AAT(P1) of the AAT values during the activity intervals of P4, P1) for the sizes of active tissue areas measured during the activity period of the respective signals. The respective visual property values are indicative of the statistical measure values AAT(P4), AAT(P1).

In other words, the AAT size values around the time point T1 are significantly smaller than for other time points during the BCL. This means that the size of the potential ablation area at locations which are active around T1 is relatively small compared to the other areas affected by the macro-reentry. To visualize this information for diagnosis support to the medically trained user the visualizer component is setting visual property values for the surface elements being associated with the sensors S1 and S4 which reflect the averaged AAT values for the corresponding activity intervals. As a result, the surface elements associated with S4 receive a visual property value reflecting AAT(P4) and the surface elements associated with S1 receive a visual property value reflecting AAT(P1). For example, a color scheme may be used, where the color spectrum from red to blue is distributed over the AAT values of the curve 610. The color value at T3 (AAT curve maximum) may be assigned to blue and the color value at T1 (AAT curve minimum) may be assigned to red. The color assigned to the averaged AAT value AAT(P4) would then be in the red part of the spectrum with a slight shift towards orange. The color for AAT(P1) would also be in the red part of the spectrum where the shift into orange exceeds the one of AAT(P4). The red colored area is then an indicator for a potential ablation area. The curve 610 may show multiple local minima dependent on the patient's heart disease. In such cases, there may be multiple areas with visual property values in the red part of the spectrum indicating various alternatives for the ablation therapy. The medically trained person finally decides where to apply the ablation therapy.

Instead of averaging, other statistical methods (e.g., median, mode, first 10-quantile, etc.) may be used to determine the statistical measures for the activity periods at the respective sensor locations. Further, other visual properties for highlighting may be used as described earlier.

In one embodiment, a threshold value may be set for the statistical measure values. In this embodiment, only the surface elements of sensor locations are highlighted as potential ablation areas where the statistical measure value of the AAT sizes is below the threshold value. All other surface elements may be associated with a default visual property value (e.g., color=blue). This embodiment provides an improved distinction of potential ablation areas from other areas.

FIG. 6B is a graph 620 illustrating the size of area of active tissue AAT over time with the minimal area of active tissue MAAT during the basic cycle length BCL. In this embodiment, only the MAAT is indicated to the medically trained person. The advantage over the embodiment of FIG. 6A is that the smallest possible area for potential ablation is indicated. The disadvantage is that only one area is indicated and no alternatives are highlighted. In this embodiment, the minimum value of the AAT curve 620 can be determined by any algorithm for the detection of minima. In the example, the minimal value MAAT is found at time point T1. In case of using again color as the visual property, the color values of all surface elements associated with sensor locations showing activity at T1 (in the example: S1, S4) are set to the color value (e.g., red) reflecting the smallest AAT value MAAT during the BCL. The visual property values of all other surface elements may be set to a different color (e.g., blue). For example, the default color value of all surface elements may be blue. The default value is then overwritten with the MAAT color value once identified by the analyzer and visualizer components. A person skilled in the art can use other visual properties to achieve a similar highlighting effect for the medically trained user.

FIG. 7 is a schematic illustration of the atrium 500 with representations of sensor locations as black bullet points and representations of the sensor locations of S1, S4 as circles. Around S1, S4 a potential ablation area 501 is indicated. In the example, the excitation wave propagating through the front side of the atrium is illustrated through the area between the dashed lines 502, 503. The dashed lines may correspond to the boundary to previously ablated or scarred tissue, which limits the area of excitable atrial tissue and prevents the atrial excitation from leaving the dashed boundary. The minimal area of active tissue is at the highlighted sensor locations S1, S4 which corresponds, in the example, to the critical isthmus. The shape of potential ablation area 501 is a schematic illustration. In a real implementation, the shape is determined by the surface texture used for a 3D visualization of the atrium 500. This is explained in detail in FIG. 8.

FIG. 8 shows a magnified portion of FIG. 7 including the potential ablation area with highlighted surface elements. Typically, 3D visualizations of three-dimensional objects use surface textures based on polygons. In the example, the surface of the 3D visualization of the atrium 500 is based on triangles 1 - 29. Other polygons can be used as well by persons skilled in the art. In the example of FIG. 8 a pattern property is used as visual property. The surface elements associated with the sensor locations S1, S4 show a dotted pattern filling whereas the other surface elements have a white filling. Once the sensor locations of S1, S4 are determined as the locations associated with the minimal area of active tissue (MAAT embodiment), the visual property values of the surface elements associated with the sensor locations are set to the dotted pattern filling.

A person skilled in the art may use various algorithms to uniquely assign each surface element to exactly one sensor location. In the example, the shortest distance of the center of gravity of a triangle surface element to the neighboring sensor locations determines to which sensor location the surface element belongs. In the example, the surface elements 1, 2, 15, 17, 18, 19, 25 are assigned to S1. The surface elements 5, 6, 8, 9, 10, 13 are assigned to S4. Instead of evaluating the shortest distance to the center of gravity, other methods (e.g., distance to closest vertices of the polygon, number of closest polygon vertices, etc.) may be used to assign the surface elements to sensor locations.

In the MAAT embodiment, both sensors show simultaneous activity during the time point T1 (cf. FIG. 6B) when the MMAT value is reached and the surface elements of both sensor locations receive the same visual property value (dotted pattern filling) reflecting the MMAT value at the time point T1. In the embodiment using statistical measures, the surface elements assigned to S1 receive a visual first property value (e.g., a first color) which is different from a second visual property value of the surface elements assigned to S4 (e.g., a second color). Thereby, the first visual property value reflects the statistical AAT measure computed for the activity interval of S1 and the second visual property value reflects the statistical AAT measure computed for the activity interval of S4.

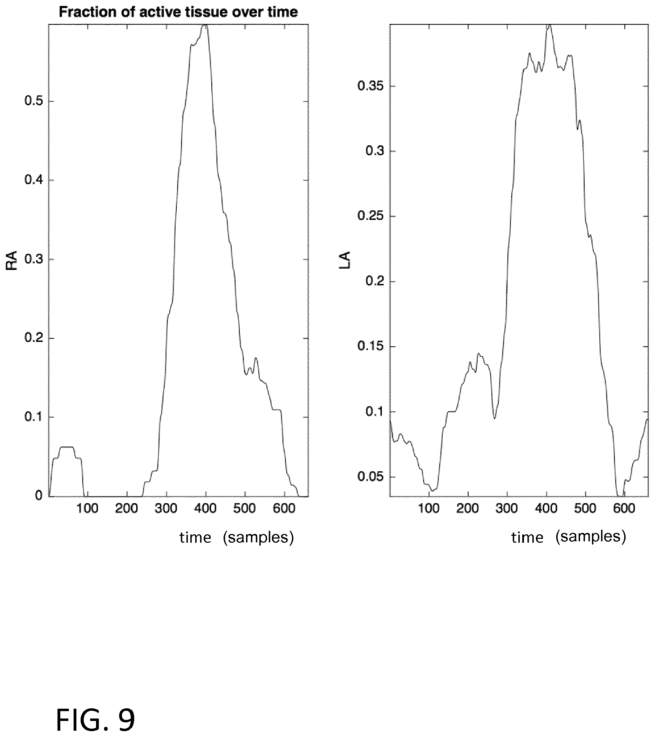

FIG. 9 shows two examples of active tissue graphs for a right and a left atrium RA, LA based on real sensor data. The sensor data was sampled at a sampling rate of 2035 Hz. The time axis in the figure has the unit of samples received. The basic cycle length in the example is approximately at 660 samples (corresponding to approximately 330 ms). The area of active tissue dimension shows the fraction of active tissue compared to the total area of tissue (i.e., the sum of all surface elements representing the surface of the atrium). For the right atrium RA this fraction reaches almost 60 % whereas for the left atrium LA the fraction only reaches almost 40 %. Between 100 samples (corresponding to 50 ms) and 230 samples (corresponding to 115 ms), the AAT curve for the RA has the value of 0, indicating that no activity was found in this atrium for the corresponding approximately 20% of BCL. Thus the presence of a reentrant mechanism can be excluded for the RA. In the LA curve for AAT, the curve does fall below 0.03, indicating that activity was present in the LA at each point in time of the BCL. This favors the presence of a reentrant atrial flutter mechanism which is located in the LA and passively causes the RA activation. The fraction visualization of the AAT curve as shown in FIG. 9 is equivalent to the absolute area size visualization as used in FIGs. 6A, 6B. Both implementations may be used with every embodiment.

Looking at the right graph for the left atrium LA, the graph shows three local minima but does not show a AAT value of zero at any time point during the BCL. In the embodiment identifying the MMAT only the minimal area of active tissue is indicated. That is, the surface elements associated with sensor locations showing activity at the point in time when the MMAT is active (approximately at 590 samples) receive a visual property value which indicates the MMAT. The other local minima at approximately 110 samples and 260 samples are not highlighted in this embodiment. Nevertheless, at least the area of active tissue associated with the first local minimum (110 samples) may be equally suitable as the MMAT.

When using the embodiment based on statistical measures at least the first local minimum (110 samples) is also highlighted with a visual property value similar to the one computed for the absolute minimum. Also the AAT associated with the second local minimum (260 samples) may be highlighted with a visual property value which indicates that the respective AAT size is larger than the AAT size associated with the other minima but, nevertheless, significantly smaller than the AAT size associated with time points between 300 samples and 500 samples. In this embodiment, a threshold value may be used to cut off the visualization of AATs above the threshold value. For example, a threshold value of 0.075 would cut off the second minimum (260 samples) and only the AATs associated with the first and third minima would be highlighted as potential ablation areas.

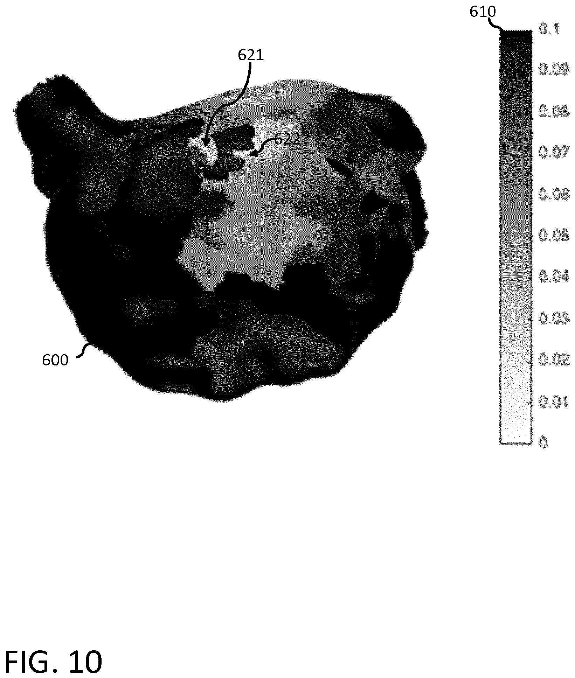

FIG. 10 shows an example of an atrium visualization 600 which is color coded with a grey scale 610 according to one embodiment to indicate potential ablation areas. The visualization is based on the embodiment using statistical measures where the surface elements associated with respective sensors receive visual property values indicating the statistical measure value (e.g., average value) for the AAT during the active period of the respective sensor signal. The grey scale values reflect fraction AAT values from 0 to 0.1. When inspecting the complete curve of fraction AAT values (not shown), all values were found to be above 0 for each time step within the BCL, indicating a reentrant atrial flutter mechanism. Manual visual inspection of the LAT map of this patient (not shown) confirmed the reentrant mechanism and revealed a zone of slow conduction in this atrium, located at the anterior wall and being in agreement with the area of FIG. 10 colored in bright grey scale values. This confirmed the role of the area showing low fraction AAT values as critical isthmus for this patient. In the example, in the upper part, two areas 621, 622 with low fraction AAT values are highlighted indicating potential ablation areas which have a comparable size smaller than the other color coded areas. Dependent on the type of the atrial flutter the medically trained person can then make a decision about the location for the ablation therapy. The atrial flutter type can be derived from inspection of the LAT map by a medically trained person, or an indication of the atrial flutter type may be derived by further functions of the computer system which are explained in the following.

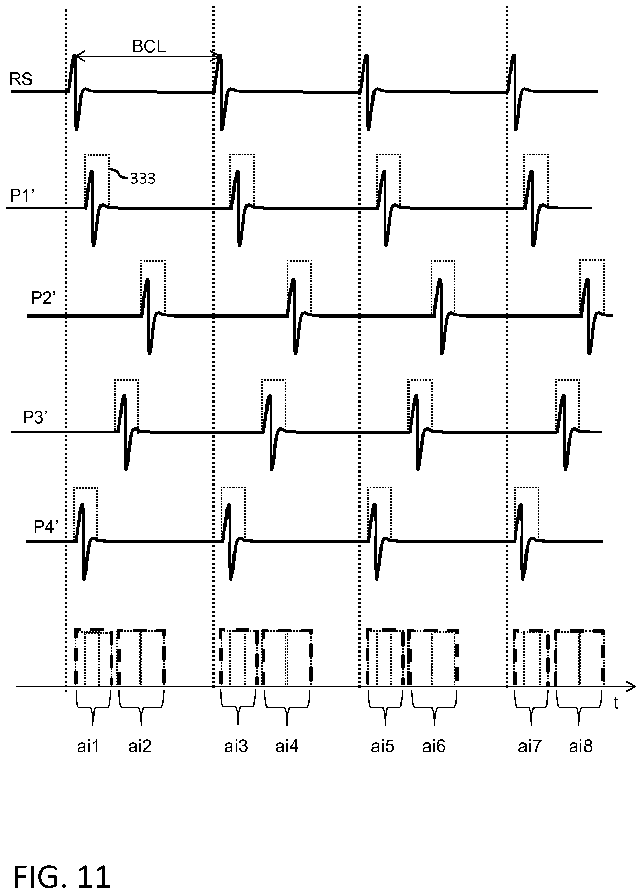

FIG. 11 illustrates sensor signals P1' to P4' received from different locations of a patient's atrium with aggregate activity periods ai1 to ai8 during the basic cycle length BCL. The sensor signals P1' to P4' may be identical to the signals P1 to P4 of FIG. 5. In this embodiment, the computer system of FIG. 1 can implement further functions to assist the medically trained person to recognize which type of atrial flutter is present in the patient's atrium.

In this embodiment, the signal analyzer component 120 (cf. FIG. 1) may determine, for each signal of at least the subset of the received signals originating from locations within the selected area, an active interval ai1 to ai8 when electrical activity occurs, and a resting interval when no electrical activity occurs. Based on the determined intervals, a subset cycle length coverage can be computed as the ratio of duration of activity (DoA) and the basic cycle length (BCL) wherein the duration of activity includes the active intervals for the entire subset of signals. The visualizer component 130 (cf. FIG. 1) can indicate the selected area as an area including a potential focal source if the subset cycle length coverage is smaller than a predefined ratio. Otherwise, the selected area is indicated as an area including a potential re-entry.

For computing the subset cycle length coverage, the analyzer component firstly determines the duration of activity for the active intervals for the entire subset of signals P1' to P4'. For example, a Boolean OR operator can be applied to the rectangular shapes (e.g., 333) representing the lengths of the determined intervals of respective signals so that any activity interval of the signals of the subset contributes to the duration of activity. To determine the overall duration of resting (across the signals of the subset). With regards to the duration of resting, only periods with no activity in all of the signals can contribute when a Boolean OR operator is applied. The bottom line of FIG. 11 illustrates the result of the OR operator applied to the signals P1' to P4'. In each cycle two activity intervals are detected which are illustrated by the dashed frames. In the first cycle, the Boolean OR application results in the activity intervals ai1, ai2. In the second cycle the result is ai3, ai4, and so forth.

Based on the determined intervals, a comparator component of the signal analyzer 120 computes a subset cycle length coverage as the ratio of duration of activity (DoA) and the basic cycle length BCL wherein the duration of activity includes the active intervals for the entire subset 121 of signals. In other words, the DoA corresponds to the total active time of the atrium in the selected area which is monitored by the sensors providing the signals of the subset 121. The comparator compares the determined subset cycle length coverage to a predefined ratio. According to studies (e.g., the study illustrated in the above cited reference "A multi-purpose spiral high-density mapping catheter..." by Jones et al.) it is known that the predefined ratio which can be used to distinguish the selected area with regards to the atrial flutter types focal source and re-entry is approximately 85%-95% (the exact number may vary according to different studies). If the selected area is an area which includes a potential focal source then the subset cycle length coverage determined from the signals of the subset 121 is smaller than said predefined ratio. If the subset cycle length coverage is equal or above said predefined ratio, the selected area is an area including a potential re-entry.

As discussed above, visual parameters, such as color, patterns or animations may be used to render a graphic representation of the patient's active atria tissue on a display device in such a way that the medically trained user of the system is provided with clear information about the potential clinical state (focal source vs. re-entry) of the patient's atria which facilitates the diagnostic analysis by the user. Any appropriate display technology (e.g., monitor, touch screen, etc.) may be used for the visualization of the atrial flutter types.

In one embodiment, an additional filter function may be used by the signal analyzer to discard signals from the subset which may be tampered with pulses from the patient's heart's ventricles. In this embodiment, a surface electrogram ECG can also be recorded simultaneously to the intracardiac electrical signals P1 to Pi, and can then be analyzed to detect ventricular depolarizations (QRS complexes). Intracardiac signals may be compromised by far fields originating from ventricular depolarizations. This may lead to electrical activity in the intracardiac signal causing deviations from baseline and thus potentially being misinterpreted as activity originating from an atrial source. Thus the activity of the atrium should be assessed while no QRS is present. The filter function may now discard or ignore any signals of the subset during such intervals where QRS is present.

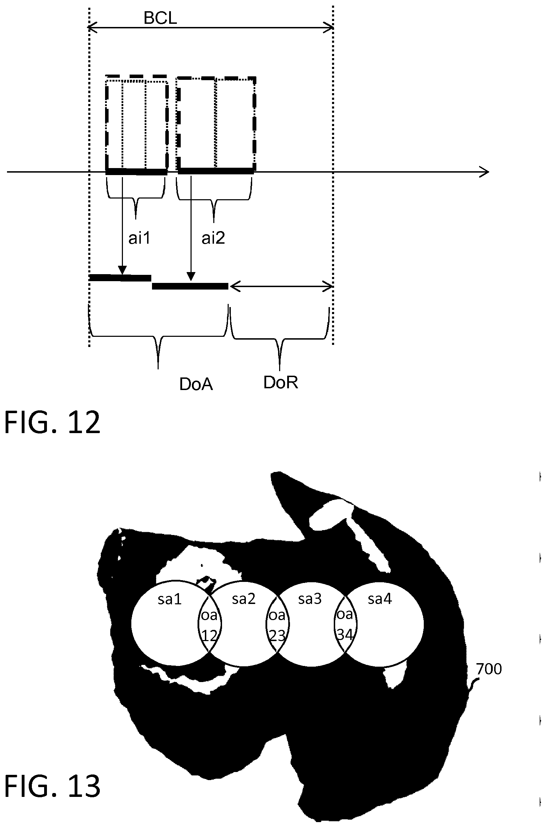

FIG. 12 illustrates the duration of activity DoA and the duration of resting DoR for the determination of cycle length coverage. The determined activity intervals ai1, ai2 of the first cycle are added to provide the overall duration of activity DoA. By computing the ratio between the duration of DoA and BCL the subset cycle length coverage is computed for the selected area which is monitored by the subset of signals P1' to P4'. It is to be noted that in a real world diagnosis scenario the number of signals in a subset can easily exceed 1000 in cases where a database is providing historic sensor data. In real time monitoring scenarios the number of simultaneously received signals is limited by the number of sensors which can be operated simultaneously in the patient's heart. It is to be noted that historic sensor data, as used herein, may have been collected very recently (e.g., a few seconds or minutes before being processed by the signal analyzer). That is, although not representing real-time data, historic sensor data may also reflect the current medical state of the patient's atria.

FIG. 13 is a simplified example of overlapping sub-areas sa1 to sa4 on a graphic representation 500 (corresponding to the selected area) of an atrium according to an embodiment of the invention.

Upon detection of a potential re-entry in the selected area (i.e., If the subset cycle length coverage is determined equal or above the predefined ratio), the signal analyzer component further selects a plurality of sub-areas sa1 to sa4 within the originally selected area 500. The sub-areas are automatically selected by the signal analyzer component according to predefined selection rules so that each sub-area has a size which is smaller than the size of the selected area 500, and each sub-area has an overlapping area oa12 to oa34 with at least one further sub-area. The predefined selection rules may include a predefined shape for sub-areas and predefined overlapping parameters. For example, in one embodiment, the selection rules may define the sub-areas as having a circular shape with a diameter between 2 and 4 cm. The sub-area dimensions relate to the respective heart portion covered by a particular sub-area (and not to the dimensions of the visualization of the heart which may be magnified or demagnified). The overlapping parameters may define that the overlapping areas between two overlapping circles account for 1/5 to 1/3 of the sub-area diameter. In an alternative embodiment, the overlapping parameters may define circle centers of two neighboring sub-areas having a distance between 0,5 cm and 1,5 cm. In another embodiment, the shape parameters may define a square shape with a diagonal between 2 cm and 4 cm, and the overlapping parameters may define square centers of two neighboring sub-areas having a distance between 0,5 cm and 1,5 cm. In an alternative embodiment, the sub-areas have a square shape and the overlapping areas between two overlapping circles account for 1/5 to 1/3 of the sub-area diagonal. A person skilled in the art can use other geometric forms for the sub-areas and achieve a comparable coverage of the selected area with a comparable resolution by applying similar overlapping ratios as disclosed for circle and square shaped sub-areas. Further, the selection rules may define particular portions of the selected area as areas of interest with regards the analysis of atrial flutter types. Such areas of interest may be received from the medically trained user or they may be predefined in the system. The signal analyzer can then distribute the sub-areas according the corresponding selection rules across the entire selected area or across one or more particular portions (areas of interest) of the selected area.

In the example of FIG. 13, for the reason of simplicity, only one row of overlapping sub-areas sa1 to sa2 with circle shapes is shown are shown as overlays to the representation 700 of an atrium. The skilled person, however, will understand that further rows may exist above and below the shown row which have further overlapping areas with the sub-areas sa1 to sa4. The selection rules in this example can define such further rows of overlapping sub-areas in such a way that the circle centers would be positioned on a vertical line defined by the intersection points of two neighboring sub-areas. In this example, each overlapping area has overlap portions where three sub-areas are overlapping. In another implementation, the selection rules can define such further rows of overlapping sub-areas in such a way that the circle centers would be positioned on a vertical line intersecting with the circle center of the respective above or below sub-area. In this implementation the overlapping area may relate to two or four sub-areas depending on the size parameters of the respective sub-areas. A person skilled in the art can define other appropriate selection rules to achieve sufficient coverage of the entire selected area or selected areas of interest with appropriate overlapping sub-areas.

The signal analyzer can then compute the respective sub-area cycle length coverage for each sub-area sa1 to sa4. The computation may be performed by the same algorithm which is used for the previous computation of the subset cycle length coverage for the selected areas. However, only the signals originating from sensors at locations covered by the respective sub-area are used for the computation of the respective sub-area cycle length coverage.

It may occur that different sub-area cycle length coverage values are computed for neighboring sub-areas. In this case, there is a conflict with regards to the cycle length coverage to be assigned to the overlapping areas oa12, oa23, oa34 which is defined as the intersection of the overlapping sub-areas sa1-sa2, sa2-sa3, and sa3-sa4, respectively. To resolve this conflict, for each overlapping area with conflicting sub-area cycle length coverages from at least two overlapping sub-areas, the signal analyzer applies one or more predefined conflict resolution rules to determine a respective overlapping area cycle length coverage based on the conflicting sub-area cycle length coverages. Different approaches for conflict resolution can be defined by different conflict resolution rules. For example, the signal analyzer may assign the conflicting sub-area cycle length coverage with the highest value to the respective overlapping area cycle length coverage as a high sensitivity to high cycle length coverage may be desired to identify potential micro-reentry atrial flutter mechanisms. In another example, it may assign the average value of the conflicting sub-area cycle length coverage values to the respective overlapping area cycle length coverage. In a further example, it may assign a weighted average value of the conflicting sub-area cycle length coverage values to the respective overlapping area cycle length coverage.

Once the sub-area cycle length coverage values and overlapping area cycle length coverage values are computed, the signal analyzer compares the computed values to an atrial flutter type threshold which may be different from the predefined ratio used by the first comparison for distinguishing between the atrial flutter types focal source and re-entry. However, the atrial flutter type threshold used by the second comparison may also be equal or similar to the predefined ratio. The atrial flutter type threshold is chosen as a threshold value which can be used to distinguish between the atrial flutter sub-types "micro-re-entry" and "macro-re-entry". Different medical studies propose different threshold values for this purpose. For example a threshold of 75% of BCL covered by the DoA within a sub-area of 2cm diameter is proposed in the previously cited work "A deductive mapping strategy for atrial tachycardia following atrial fibrillation ablation: importance of localized reentry" by Jais et al.. A cycle length coverage value below the atrial flutter type threshold is seen as an indicator for a potential "macro-re-entry". A cycle length coverage value equal to or above the atrial flutter type threshold is seen as an indicator for a potential "micro-re-entry".

The signal analyzer provides the result of the comparison regarding each sub-area sa1 to sa4 and each overlapping area oa12, oa23, oa34 to the visualizer component which can render a visualization of the sub-areas and overlapping areas accordingly. The rendering is performed in such a way that it indicates to the medically trained person a potential micro-re-entry if the respective cycle length coverage value is equal to or above the atrial flutter type threshold. Further, the rendering may indicate a potential macro-re-entry if the respective cycle length coverage value is below the atrial flutter type threshold (as described above). The indication is implemented via similar visualization parameters as disclosed already above for the rendering of the selected area. That is, different colors, patterns, animations, 3D-effects, etc. may be used to graphically distinguish areas with potential micro-re-entries from areas with potential macro-re-entries for the medically trained user to support the diagnostic decision making.

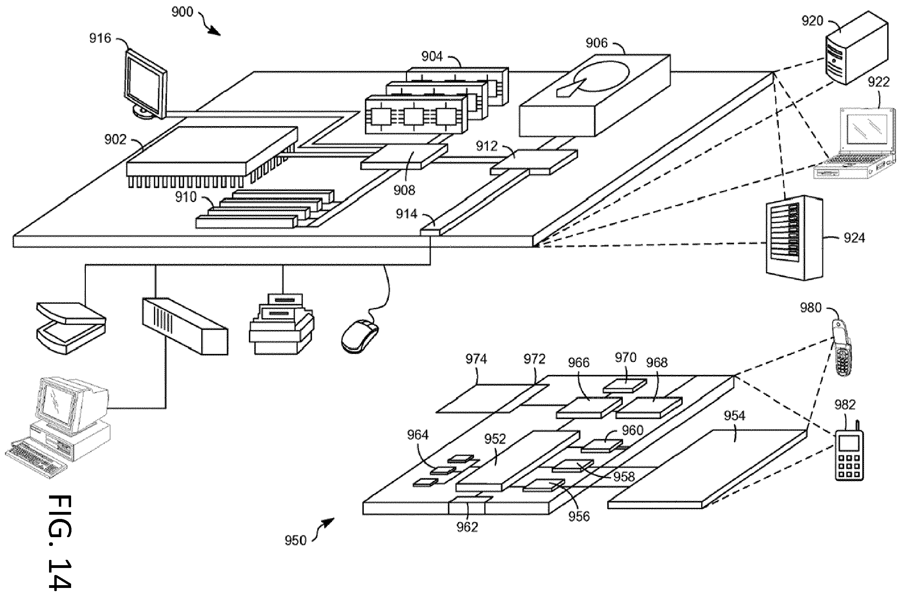

FIG. 14 is a diagram that shows an example of a generic computer device 900 and a generic mobile computer device 950, which may be used with the techniques described here. Computing device 900 is intended to represent various forms of digital computers, such as laptops, desktops, workstations, personal digital assistants, tablets, servers, blade servers, mainframes, and other appropriate computers. Generic computer device 900 may correspond to a computer system 100 as illustrated in FIG. 1. Computing device 950 is intended to represent various forms of mobile devices, such as personal digital assistants, cellular telephones, smart phones, and other similar computing devices. For example, computing device 950 may be used by a user as a front end to interact with the computer system 100. Computing device may, for example, include the display device 220 of FIG. 1. The components shown here, their connections and relationships, and their functions, are meant to be exemplary only, and are not meant to limit implementations of the inventions described and/or claimed in this document.

Computing device 900 includes a processor 902, memory 904, a storage device 906, a high-speed interface 908 connecting to memory 904 and high-speed expansion ports 910, and a low speed interface 912 connecting to low speed bus 914 and storage device 906. Each of the components 902, 904, 906, 908, 910, and 912, are interconnected using various busses, and may be mounted on a common motherboard or in other manners as appropriate. The processor 902 can process instructions for execution within the computing device 900, including instructions stored in the memory 904 or on the storage device 906 to display graphical information for a GUI on an external input/output device, such as display 916 coupled to high speed interface 908. In other implementations, multiple processing units and/or multiple buses may be used, as appropriate, along with multiple memories and types of memory. Also, multiple computing devices 900 may be connected, with each device providing portions of the necessary operations (e.g., as a server bank, a group of blade servers, or a processing device).

The memory 904 stores information within the computing device 900. In one implementation, the memory 904 is a volatile memory unit or units. In another implementation, the memory 904 is a non-volatile memory unit or units. The memory 904 may also be another form of computer-readable medium, such as a magnetic or optical disk.

The storage device 906 is capable of providing mass storage for the computing device 900. In one implementation, the storage device 906 may be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product may also contain instructions that, when executed, perform one or more methods, such as those described above. The information carrier is a computer- or machine-readable medium, such as the memory 904, the storage device 906, or memory on processor 902.

The high speed controller 908 manages bandwidth-intensive operations for the computing device 900, while the low speed controller 912 manages lower bandwidth-intensive operations. Such allocation of functions is exemplary only. In one implementation, the high-speed controller 908 is coupled to memory 904, display 916 (e.g., through a graphics processor or accelerator), and to high-speed expansion ports 910, which may accept various expansion cards (not shown). In the implementation, low-speed controller 912 is coupled to storage device 906 and low-speed expansion port 914. The low-speed expansion port, which may include various communication ports (e.g., USB, Bluetooth, Ethernet, wireless Ethernet) may be coupled to one or more input/output devices, such as a keyboard, a pointing device, a scanner, or a networking device such as a switch or router, e.g., through a network adapter.

The computing device 900 may be implemented in a number of different forms, as shown in the figure. For example, it may be implemented as a standard server 920, or multiple times in a group of such servers. It may also be implemented as part of a rack server system 924. In addition, it may be implemented in a personal computer such as a laptop computer 922. Alternatively, components from computing device 900 may be combined with other components in a mobile device (not shown), such as device 950. Each of such devices may contain one or more of computing device 900, 950, and an entire system may be made up of multiple computing devices 900, 950 communicating with each other.

Computing device 950 includes a processor 952, memory 964, an input/output device such as a display 954, a communication interface 966, and a transceiver 968, among other components. The device 950 may also be provided with a storage device, such as a microdrive or other device, to provide additional storage. Each of the components 950, 952, 964, 954, 966, and 968, are interconnected using various buses, and several of the components may be mounted on a common motherboard or in other manners as appropriate.

The processor 952 can execute instructions within the computing device 950, including instructions stored in the memory 964. The processor may be implemented as a chipset of chips that include separate and multiple analog and digital processing units. The processor may provide, for example, for coordination of the other components of the device 950, such as control of user interfaces, applications run by device 950, and wireless communication by device 950.

Processor 952 may communicate with a user through control interface 958 and display interface 956 coupled to a display 954. The display 954 may be, for example, a TFT LCD (Thin-Film-Transistor Liquid Crystal Display) or an OLED (Organic Light Emitting Diode) display, or other appropriate display technology. The display interface 956 may comprise appropriate circuitry for driving the display 954 to present graphical and other information to a user. The control interface 958 may receive commands from a user and convert them for submission to the processor 952. In addition, an external interface 962 may be provided in communication with processor 952, so as to enable near area communication of device 950 with other devices. External interface 962 may provide, for example, for wired communication in some implementations, or for wireless communication in other implementations, and multiple interfaces may also be used.

The memory 964 stores information within the computing device 950. The memory 964 can be implemented as one or more of a computer-readable medium or media, a volatile memory unit or units, or a non-volatile memory unit or units. Expansion memory 984 may also be provided and connected to device 950 through expansion interface 982, which may include, for example, a SIMM (Single In Line Memory Module) card interface. Such expansion memory 984 may provide extra storage space for device 950, or may also store applications or other information for device 950. Specifically, expansion memory 984 may include instructions to carry out or supplement the processes described above, and may include secure information also. Thus, for example, expansion memory 984 may act as a security module for device 950, and may be programmed with instructions that permit secure use of device 950. In addition, secure applications may be provided via the SIMM cards, along with additional information, such as placing the identifying information on the SIMM card in a non-hackable manner.

The memory may include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 964, expansion memory 984, or memory on processor 952, that may be received, for example, over transceiver 968 or external interface 962.

Device 950 may communicate wirelessly through communication interface 966, which may include digital signal processing circuitry where necessary. Communication interface 966 may provide for communications under various modes or protocols, such as GSM voice calls, SMS, EMS, or MMS messaging, CDMA, TDMA, PDC, WCDMA, CDMA2000, or GPRS, EDGE, UMTS, LTE, among others. Such communication may occur, for example, through radio-frequency transceiver 968. In addition, short-range communication may occur, such as using a Bluetooth, WiFi, or other such transceiver (not shown). In addition, GPS (Global Positioning System) receiver module 980 may provide additional navigation- and location-related wireless data to device 950, which may be used as appropriate by applications running on device 950.

Device 950 may also communicate audibly using audio codec 960, which may receive spoken information from a user and convert it to usable digital information. Audio codec 960 may likewise generate audible sound for a user, such as through a speaker, e.g., in a handset of device 950. Such sound may include sound from voice telephone calls, may include recorded sound (e.g., voice messages, music files, etc.) and may also include sound generated by applications operating on device 950.

The computing device 950 may be implemented in a number of different forms, as shown in the figure. For example, it may be implemented as a cellular telephone 980. It may also be implemented as part of a smart phone 982, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" and "computer-readable medium" refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing device that includes a backend component (e.g., as a data server), or that includes a middleware component (e.g., an application server), or that includes a front end component (e.g., a client computer having a graphical user interface or a Web browser through which a user can interact with an implementation of the systems and techniques described here), or any combination of such backend, middleware, or frontend components. The components of the system can be interconnected by any form or medium of digital data communication (e.g., a communication network). Examples of communication networks include a local area network ("LAN"), a wireless local area network ("WLAN"), a wide area network ("WAN"), and the Internet.

The computing device can include clients and servers. A client and server are generally remote from each other and typically interact through a communication network. The relationship of client and server arises by virtue of computer programs running on the respective computers and having a client-server relationship to each other.

A number of embodiments have been described. Nevertheless, it will be understood that various modifications may be made without departing from the spirit and scope of the invention.

In addition, the logic flows depicted in the figures do not require the particular order shown, or sequential order, to achieve desirable results. In addition, other steps may be provided, or steps may be eliminated, from the described flows, and other components may be added to, or removed from, the described systems. Accordingly, other embodiments are within the scope of the following claims.

## Claims

1. A decision support computer system (100) for supporting diagnostic analysis of potential ablation areas to cure atrial flutter, comprising:
an interface component (110) configured to receive an electrical reference signal (RS) generated by a reference sensor (CSCS), the reference signal (RS) providing a time reference for electrical activity of one or more atria of a patient wherein the time interval between two subsequent electrical activities is referred to as basic cycle length (BCL), and further configured to receive a plurality of electrical signals (P1 to Pi) generated by one or more further sensors (S1 to Sn) wherein the plurality of electrical signals (P1 to Pi) relate to a plurality of locations in the one or more atria, the plurality of locations comprising locations different from the location of the reference sensor (CSCS);
a signal analyzer component (120) configured to determine, for each signal of at least a subset of the received signals originating from locations within a selected area of the one or more atria, when electrical activity occurs, and to determine, for a plurality of time points within the basic cycle length, size values for respective areas of active tissue by assessing the aggregate size of surface elements associated with the locations where the respective signals show activity at the respective time points; and
a visualizer component (130) configured to set a visual property value for at least a subset of the surface elements wherein the subset includes surface elements associated with locations identifying a particular area of active tissue which is smaller than active tissue areas associated with other locations, the visual property value indicating, in a visualization of the selected area, the particular area of active tissue as a potential ablation area to cure atrial flutter.

2. The system of claim 1, wherein in case of a focal source atrial flutter the potential ablation area corresponds to a spot defining the approximate origin of the focal source, or in case of a micro-reentry atrial flutter the potential ablation area corresponds to a bridging area between the approximate center of the micro-reentry affected area and the closest anatomical obstacle, or in case of a macro-reentry atrial flutter the potential ablation area corresponds to the critical isthmus within the macro-reentry affected area.

3. The system of claim 1 or 2, wherein the selected area corresponds to an atrium.

4. The system of any of previous claims, wherein the visual property is selected from the group of: a particular color for highlighting the respective surface elements, a particular texture for highlighting the respective surface elements, and a particular animation mode for highlighting the respective surface elements.

5. The system of any of the previous claims, wherein the particular area of active tissue is computed as the minimal area of active tissue at a respective time point within the basic cycle length, the respective visual property value being indicative of the minimal area.

6. The system of any of the claims 1 to 4, wherein the particular area of active tissue is computed by as a statistical measure for the sizes of active tissue areas measured during the activity period of a respective signal, the respective visual property value being indicative of the statistical measure value.

7. The system of any of the previous claims, wherein
the signal analyzer component (120) is further configured to determine, for each signal of at least the subset of the received signals originating from locations within the selected area (500), an active interval (ai1 to ai8) when electrical activity occurs, and a resting interval when no electrical activity occurs; and configured to compute, based on the determined intervals, a subset cycle length coverage as the ratio of duration of activity (DoA) and the basic cycle length (BCL) wherein the duration of activity includes the active intervals for the entire subset of signals; and
the visualizer component (130) further configured to indicate the selected area as an area including a potential focal source if the subset cycle length coverage is smaller than a predefined ratio, and else to indicate the selected area as an area including a potential re-entry.

8. A computer-implemented method (1000) for supporting diagnostic analysis of potential ablation areas to cure atrial flutter, the method comprising:
receiving (1100) an electrical reference signal generated by a reference sensor, the reference signal providing a time reference for electrical activity of one or more atria of a patient wherein the time interval between two subsequent electrical activities is referred to as basic cycle length;
receiving (1200) a plurality of electrical signals generated by one or more further sensors wherein the plurality of electrical signals relate to a plurality of locations in the one or more atria, the plurality of locations comprising locations different from the location of the reference sensor;
determining (1300), for each signal of at least a subset of the received signals originating from locations within a selected area of the one or more atria, when electrical activity occurs;
determining (1400), for a plurality of time points within the basic cycle length, size values for respective areas of active tissue by assessing the aggregate size of surface elements associated with the locations where the respective signals show activity at the respective time points;
setting (1500) a visual property value for at least a subset of the surface elements wherein the subset includes surface elements associated with locations defining a particular area of active tissue which is smaller than active tissue areas associated with other locations, to indicate, in a visualization of the selected area, the particular area of active tissue as a potential ablation area to cure atrial flutter.

9. The method of claim 8, wherein in case of a focal source atrial flutter the potential ablation area corresponds to a spot defining the approximate origin of the focal source, or in case of a micro-reentry atrial flutter the potential ablation area corresponds to a bridging area between the approximate center of the micro-reentry affected area and the closest anatomical obstacle, or in case of a macro-reentry atrial flutter the potential ablation area corresponds to the critical isthmus within the macro-reentry affected area.

10. The method of any of the claims 8 to 9, wherein the visual property is selected from the group of: a particular color for highlighting the respective surface elements, a particular texture for highlighting the respective surface elements, and a particular animation mode for highlighting the respective surface elements.

11. The method of any of the claims 8 to 10, wherein the particular area of active tissue is computed as the minimal area of active tissue at a respective time point within the basic cycle length, the respective visual property value being indicative of the minimal area.

12. The method of any of the claims 8 to 10, wherein the particular area of active tissue is computed as statistical measure for the sizes of active tissue areas measured during the activity period of a respective signal, the respective visual property value being indicative of the statistical measure value.

13. The method of claim 12, wherein the statistical measure is determined by any one statistical method of the group of: arithmetic averaging, median, mode, and first 10-quantile.

14. The method of any of the claims 8 to 13, further comprising:
determining, for each of the signals of at least the subset, an active interval when electrical activity occurs, and a resting interval when no electrical activity occurs;
based on the determined intervals, computing a subset cycle length coverage as the ratio of duration of activity (DoA) and the basic cycle length (BCL) wherein the duration of activity (DoA) includes the active intervals for the entire subset of signals;
if the subset cycle length coverage is smaller than a predefined ratio then indicating the selected area as an area including a potential focal source, else indicating the selected area as an area including a potential re-entry.

15. A computer program product for supporting diagnostic analysis of potential ablation areas to cure atrial flutter, the computer program product when loaded into a memory of a computing device and executed by at least one processor of the computing device executes the steps of the computer-implemented method according to any one of the claims 8 to 14.
